Europäisches Patentamt

European Patent Office

Office européen des brevets

(11) Publication number: **0 358 500**
**A1**

## EUROPEAN PATENT APPLICATION

(21) Application number: **89309061.3**

(22) Date of filing: **07.09.89**

(51) Int. Cl.⁵: **A 61 K 37/54**
**A 61 K 37/547**

(30) Priority: **08.09.88 GB 8821049**

(43) Date of publication of application:
**14.03.90 Bulletin 90/11**

(84) Designated Contracting States: **ES GR**

(71) Applicant: **The Public Health Laboratory Service Board**
**61 Colindale Avenue**
**London NW9 5EQ (GB)**

**RETROSCREEN LIMITED**
**64 Turner Street**
**London E1 2AD (GB)**

(72) Inventor: **Sutton, Peter Morgan**
**"Manderley" Bower Gardens**
**Salisbury Wiltshire SP1 2RL (GB)**

**Oxford, John Sydney**
**70 Holden Road**
**Woodside Park London N12 7DY (GB)**

(74) Representative: **Ritter, Stephen David et al**
**Mathys & Squire 10 Fleet Street**
**London EC4Y 1AY (GB)**

(54) **Method and composition for the treatment and prevention of viral infections.**

(57) The invention provides a method of treating a viral infection in a subject, which comprises administering a protease enzyme, especially a plant protease such as, for example a bromelain, papain or ficin. Examples of viral infections which may be treated include infections with DNA- or RNA-containing viruses or infections caused by prions.

The invention further provides the use of proteases in the production or sterilization of pharmaceutical preparations or food-stuffs in order to reduce the content of virus, pro-virus or virus-infected cells therein and the use of proteases in the sterilization or decontamination of surgical devices. Production of inactivated or attenuated virus for incorporation into a vaccine or vaccine component for providing a protective effect against viral infection in humans or animals is also described.

**Description**

# METHOD AND COMPOSITION FOR THE TREATMENT AND PREVENTION OF VIRAL INFECTIONS

This invention relates to a method and composition for the treatment and prevention of viral infections.

The invention is of particular utility in the treatment and prevention of infections with enveloped viruses including, for example human immuno-deficiency viruses (HIV) and influenza viruses.

Acquired Immune Deficiency Syndrome (AIDS) has been described as "a modern plague: the first great pandemic of the second half of the twentieth century". The quest for an effective therapy for AIDS has become a major priority for research, but despite a massive commitment of resources, few drugs are available for therapy, all have significant side effects and none is curative.

The causative agent of AIDS is a retrovirus previously referred to as HTLV-III or LAV, but now referred to as HIV. Current therapies proposed for combatting HIV infections generally involve the use of anti-viral agents which interfere with viral replication, for example by inhibiting reverse transcriptase. Such agents are generally cytotoxic and despite the limited success which has been achieved with such anti-viral agents as zidovudine (AZT), no effective treatment of AIDS has been found to date.

The present invention is based on the discovery that proteolytic enzymes (proteases) may be used in the therapy of viral infections. This is unexpected in that enzymes play only a minor role as therapeutic drugs, for example streptokinase is used as a fibrinolytic agent while asparaginase forms part of most combination therapies for childhood acute lymphatic leukaemia. Certain enzymes have been administered orally in an attempt to reduce soft tissue inflammation, notably the bromelains, which are proteases obtained from pineapple plants. However the British National Formulary emphasises that their therapeutic effectiveness is doubtful. An available dosage form of bromelains has until recently been marketed by Rorer Pharmaceuticals (a subsidiary of Fisons Limited) under the proprietory brand name "Ananase Forte". Bromelains have further been used in skin care lotions, for promoting healing and have also been proposed as plasminogen activators in the treatment of thrombo-occlusive vascular disease. There has never, however, been any suggestion of the use of bromelains or other proteases as antiviral agents.

Thus according to one aspect of the present invention there is provided a method of treating a viral infection in a subject, which comprises administering a protease.

The invention further provides the use of a protease in the production of a pharmaceutical composition for use in treatment of a viral infection in a subject.

The term "virus" as used herein is intended to include any entity capable of infecting a eukaryotic cell, whereby the infected cell can produce duplicates of the infectious entity. Thus the term "virus" embraces DNA- or RNA-based infectious particles where the nucleic acid is associated with other macromocular species such as, for example, proteins and lipids. The term also includes so-called "prions" which are infectious entities implicated in such diseases as scrapie in sheep, bovine spongiform encephalopathy in cattle, and Kuru and Creutzfeltd-Jakob disease in man.

The method of the invention may be applied to the prevention and treatment of a wide range of viral infections. Examples include infections with retroviruses such as HIV referred to previously, and with other enveloped viruses such as, e.g. influenza viruses. Such enveloped viruses usually have surface glycoprotein "spikes" (peplomers).

A further application of proteases in accordance with the invention comprises the production of vaccines for providing a protective effect against viral infection in humans or animals. In accordance with this aspect of the invention there is provided a method for the production of inactivated or attenuated virus for incorporation into a vaccine or vaccine component which comprises contacting said virus with a protease.

Other applications of proteases in accordance with the invention include the use of proteases in the treatment of a food product which is contaminated with virus or suspected of having such contamination, which comprises contacting said food product with said protease. Examples of such food products include meat suspected of being contaminated with viruses or with the causative agents (prions) responsible for scrapie in sheep and bovine spongiform encephalopathy in cattle.

A wide range of proteases may be used in carrying out the method of the invention and in general, the enzyme of choice will be determined by the nature of the viral infection being treated and the required route of administration. Thus, for example, where the nature of the viral infection makes it necessary for the protease to be introduced into the systemic circulation, the selected protease should have a relatively low immunogenicity and/or toxicity. It is also advantageous in such circumstances to be able to use a protease which is capable of entering the systemic circulation following administration of a suitable dosage form.

Generally, proteases of non-animal origin are preferably used in accordance with the invention. Thus, it is particularly preferred to use a plant protease such as, for example, a bromelain, papain or ficin. Processes for producing these proteases are described in US Patent Nos 2950227 and 3002891. The latter specifically relates to the extraction of bromelains from pineapple stems. However both stem and fruit bromelains may be used in the method of the invention.

Other proteases useful in accordance with the invention include digestive enzymes such as, for example trypsin and chymotrypsin.

In carrying out the method of the invention, the protease may be administered by any convenient means. Thus, for example, it may be administered orally or parenterally, or in the case of a local viral infection, it may be administered topically. Parenteral modes of administration include administration by injection intravenously, intramuscularly, subcutaneously, intrathecally or into a body cavity, e.g. intraperitoneally or intrapleurally.

Advantageously, the protease is administered orally in which case, in order to avoid inactivation in the stomach, the protease is preferably administered in the form of an enteric-coated tablet or pill, or in the form of a capsule resistant to degradation in the stomach.

Other methods of administration may be adopted, for example rectal administration in the form of a suppository, in the form of an aerosol as in a nasal spray or as eye drops.

A suitable dose is dependent on the mode of application, the toxicity of the selected protease and the proteolytic activity of the protease. For specific combinations of these parameters, effective dosage ranges may be determined by carrying out appropriate *in vitro* and *in vivo* experiments. Generally it has been found that concentrations of proteases in the range 1-100 μg/ml, particularly 5-100 μg/ml can exert an anti-viral effect and dosages capable of producing such levels in the blood-stream are advantageously employed.

Proteases may be used in accordance with the present invention in combination therapies together with other therapeutic agents having an antiviral activity. Thus, for example, in the treatment or prevention of HIV infections, combination therapies involving the simultaneous or successive administration of a protease and zidovudine (AZT) are included within the scope of the present invention. A particularly useful combination therapy which does not require the use of zidovudine or related nucleoside analogues comprises administering, simultaneously or successively, a protease in accordance with the present invention and a surfactant and/or a steroid in accordance with the procedures described in our European Patent Application No. 85302275.8 (EP-0285285).

The compositions produced in accordance with the invention may comprise conventional pharmaceutically acceptable diluents or carriers. Thus typical injectable solutions will comprise sterile pyrogen-free media, e.g. normal saline and optionally include buffering agents, stabilising agents and preservatives. Similarly, conventional enteric coatings may be used. Tablets and pills may include conventional excipients such as, for example, lactose, starch and magnesium stearate while suppositories will include excipients such as waxes and glycerol.

Other examples of compositions according to the invention are topical compositions for treating and/or protecting a subject against viral infection. Such compositions may comprise a protease and an excipient adapted for topical application. Such compositions may for example be in the form of creams, ointments, lotions, solutions or gels. Topical compositions in forms suitable for vaginal use (e.g. creams, ointments, gels, foams and soluble pessaries) can offer protection against transmission of viral infections during sexual intercourse. These compositions may advantageously contain a spermicidally active agent.

In accordance with a further aspect of the invention, a protease may be used in the production or sterilisation of pharmaceutical preparations (particularly injectable pharmaceutical preparations) and surgical devices in order to reduce the content of (or contamination with) infectious virus, pro-virus, prion or virus-infected cells therein. By the term "pharmaceutical preparation" as used herein is meant any substance, composition, living tissue, implantable material or prosthetic device useful in medical treatment or therapy. Similarly "surgical device" is intended to include any surgical apparatus or instrument, or any product or device used in a surgical treatment, including, e.g. a suture or catheter.

Examples of pharmaceutical preparations include whole blood (for transfusion), blood plasma or other blood products (e.g. Factor VIII). For use in accordance with the invention, the protease may be added so as to produce a concentration sufficient to inactivate any virus present. The concentration should not be so high, or the treatment sufficiently long to cause substantial deterioration of the product being treated.

Proteases may be used in accordance with the invention for treating tissues for use as grafts and transplants, in order to reduce the likelihood of transmission of viral infections. This is particularly important in the transplantation of e.g. corneal tissue, or in transplant techniques in which living cells are brought into contact with nerve cells. Thus in the past, individuals have inadvertently been infected with the infectious agent of Creutzfeldt-Jakob disease by transplantation of infected corneal tissue.

A further utility of proteases in accordance with the invention comprises applying a protease to a food product in order to eliminate virus particles therefrom. Thus, for example, meat products may be treated with proteases in accordance with the invention in order to eliminate contamination with virus. This procedure is particularly useful in eliminating prions from animal-derived foodstuff destined for human or animal consumption.

The following pharmacological data, given by way of example, illustrates the present invention.

## 1. ANTIVIRAL ACTIVITY

### 1.1 Influenza Viruses

The antiviral activity of bromelain on three strains of influenza virus was assessed according to the following protocol. The bromelain used in this and subsequent experiments was obtained from Siber Hegner Limited and had the following characteristics:

Lot No. : 901026
Activity: 1200 GDU/g
Ash : 2.67%
Moisture: 2.6%

Samples of allantoic fluid infected with influenza virus were mixed with varying concentrations of bromelain and incubated at 37°C for 1 hour in phosphate buffered saline (PBS) or in broth saline at pH 7.0. Two strains of influenza A virus (HINI and H3N2) and one strain of influenza B virus (B/Yamagata/10/88) were used in the experiments.

The incubated fluids were then titrated for residual virus infection using 10 day-old embryonated hen's eggs by injecting samples into the eggs using standard techniques.

Injected eggs were incubated for 2 days at 37°C, chilled at 4°C and allantoic fluid removed and tested for virus haemagglutinin using 0.5% turkey erythrocytes.

The results are given in the following Tables 1, 2 and 3.

## TABLE 1

Effect of Bromelain on Influenza A (H3N2) Virus Infectivity

| Concentration of drug μg/ml | Titre of Virus ($\log_{10}$ $EID_{50}$/ml)* | Reduction in Titre ($\log_{10}$ $EID_{50}$/ml) |
|---|---|---|
| 1000 | 6.0 | 3.2 |
| 100 | 8.0 | 1.2 |
| 0 | 9.2 | 0.0 |

Virus incubated in broth saline 1 hour at 37°C.

* $EID_{50}$/ml = Egg infective Dose$_{50}$ expressed in ml.

## TABLE 2

Effect of Bromelain on Influenza A (H3N2) Virus Infectivity

| Concentration of drug μg/ml | Titre of Virus ($\log_{10}$ $EID_{50}$/ml)* | Reduction in Titre ($\log_{10}$ $EID_{50}$/ml) |
|---|---|---|
| 100 | 5.2 | 1.0 |
| 0 | 6.2 | 0.0 |

Virus incubated in PBS 1 hour at 37°C.

* $EID_{50}$/ml = Egg infective Dose$_{50}$ expressed in ml.

## TABLE 3

Effect of Bromelain on Influenza A (H1N1) and B Virus Infectivity

| Concentration of drug μg/ml | Titre of Virus ($\log_{50}$ $EID_{50}$/ml) | Reduction in Titre ($\log_{10}$ $EID_{50}$/ml) |
|---|---|---|
| B/Yamagata/ 10/88 | | |
| 100 | 8.5 | 1.0 |
| 0 | 7.5 | 0.0 |
| A/England/ 427/88 (H1N1) | | |
| 100 | 5.9 | 1.3 |
| 0 | 7.2 | 0.0 |

It can be seen from the results in the above tables that a significant decrease in virus infectivity was detected both with influenza A and B virus after incubation for 1 hour at 37°C with concentrations of bromelain of 10 μg/ml and above. Further, the results in Tables 1 and 2 demonstrate that the inhibitory effect was not abrogated by protein in the culture medium.

These results are in contrast to the anti-viral effect of the known anti-influenza drug amantadine, which is effective against influenza A, but not influenza B.

### 1.2 HIV Virus

#### 1.2.1 H9 III B Cell Line With C8166 Marker

The antiviral activity of bromelain on HIV was assessed using the persistently HIV-infected human T-lymphocytes cell line known as "H9" (more specifically the H9 III B cell line). Uninfected cells (an established human T-cell line, specifically the C8166 cell line) were used as markers.

The H9 cells, at a concentration of $2 \times 10^5$ cells/ml, were incubated in wells of a 24 well microtitre dish (0.5 ml/well) in RPM1 1640 medium (RPMI 1640 medium is a complex mixture of amino acids, salts, glucose and vitamins, obtainable from Gibco BRL under Catalogue Ref. 041-02400). Bromelain stock solution was serially diluted to concentrations of 200, 20, 2.0 and 0.2 μg/ml, and 0.5 ml aliquots were added to the wells to give final concentrations of 100, 10, 1.0 and 0.1 μg/ml respectively.

1 ml aliquots of uninfected C8166 cells at a concentration of $10^5$ cells/ml or of culture media alone were added to the wells and the dish was incubated overnight at 37°C in an atmosphere of 5% $CO_2$/95% air.

200 μl samples of supernatant were removed on Day 1, diluted 1:4 and assayed for HIV using the standard Coulter Antigen (P24) ELISA test, having an automated Optical Density (O.D.) system.

The results are shown in Figure 1a.

#### 1.2.2. H9 III B Cell Line Without Marker Cells

Experiment 1.2.1 was repeated, but omitting the C8166 marker cell line.

The H9 cells at a concentration of $2 \times 10^5$ cells/ml

were incubated in wells of a 24 well microtitre dish. Serial dilutions of bromelain stock solution was added to give a final concentration in the wells of 50, 5.0, 0.5 and 0.05 µg/ml.

Thereafter the protocol was as described in Experiment 1.2.1.

The results are shown in Figure 1b.

1.2.3 Results

It is apparent that an inhibitory effect on production of virus antigen (p24) was detected both in the persistently infected H9 cells (H9IIIB) and in the combination of co-infected C8166 cells and H9 cells. The virus inhibiting effect was particularly significant at a bromelain concentration of between 0.5 µg/ml and 50 µg/m.

2.TOXICITY

The toxicity of bromelain to CEM cells, C8166 cells and cord blood lymphocytes was determined in the following manner for each cell line.

A suspension of the cells in RPM1 1640 with hepes and 1-glut + 3% FCS (plus interleukin 2 for the cord blood lymphocytes) was centrifuged and the cell pellet resuspended in the same medium at a concentration of $10^6$ cells/ml 0.75 ml aliquots of the suspension were placed in wells of 24-well tissue culture plate and 0.75 ml of bromelain solution added to selected wells. The following concentrations of bromelain solution were used in the experiment:
1000 µg/ml
100 µg/ml
1.0 µg/ml
0.01 µg/ml
and also a bromelain-free control.

At various intervals (e.g. 24 hours, 4 and 6 day intervals) 50 µl samples were removed and 50 µl of trypan blue added. The total number of viable cells was counted, by failure to be stained by the dye.

The results are shown in Figure 2.

3. CONCLUSIONS

From the data presented, the following conclusions can be drawn.

3.1 Anti-influenza Effect

A significant decrease in virus infectivity was detected both with influenza A and B viruses after incubation for 1 hour at 37°C with concentrations of 100 µg/ml or greater of bromelain (Tables 1-3).

3.2 Anti-HIV Effect

An inhibitory effect on production of virus antigen (p24) was detected both in persistently infected H9 cells (H9 IIIB) and in co-incubated C8166 cells and H9 IIIB (Figures 1a and 1b). This virus inhibitory effect was detected at bromelain concentrations exceeding 0.5 µg/ml.

3.3 Cell Toxicity

Experiments with human cord lymphocytes have demonstrated the relatively low toxicity of bromelain, particularly at the levels shown to exhibit an anti-viral effect. Concentrations of bromelain up to and including 25 µg/ml exhibited no toxic effects on dividing cells after 6 days incubation at 37°C. After 4 days incubation concentrations up to 250 µg/ml were tolerated.

The two established cell lines CEM and C8166 were somewhat more sensitive to bromelain if cultured for 4 days or longer.

3.4 General

Although the precise mechanism responsible for the anti-viral activity of proteases is not known, it is postulated that the protease acts by removing the glycoprotein spikes (peplomers) which are characteristic of enveloped viruses, leaving the resulting bald particles non-infectious.

**Claims**

1. A method of treating a viral infection in a subject, which comprises administering a protease enzyme.

2. A method according to Claim 1 wherein the protease is capable of entering the systemic circulation following administration of a suitable dosage form.

3. A method according to Claim 1 or Claim 2 wherein the protease is of non-animal origin.

4. A method according to Claim 3 wherein the protease is a plant protease.

5. A method according to Claim 4 wherein the protease is a bromelain, papain or ficin.

6 A method according to Claim 1 or Claim 2 wherein the protease is of animal origin.

7. A method according to Claim 6 wherein the protease is selected from trypsin and chymotrypsin.

8. A method according to any preceding claim wherein the the protease is administered orally

9. A method according to Claim 8 wherein the protease is administered in the form of an enteric-coated tablet or pill or in the form of a capsule resistant to degradation in the stomach.

10. A method according to any of Claims 1 to 7 wherein the protease is administered parenterally.

11. A method according to any of Claims 1 to 7 wherein the protease is administered topically.

12. A method according to any preceding claim wherein said viral infection is an infection with a DNA- or RNA-containing virus or an infection caused by a prion.

13. A method according to Claim 12 wherein said viral infection is an infection with an enveloped virus.

14. A method according to Claim 13 wherein the enveloped virus is HIV or influenza virus.

15. The use of a protease enzyme in the production of a pharmaceutical composition for use in treatment of a viral infection in a subject.

16. The use claimed in Claim 15 wherein the treatment comprises a method according to any of Claims 2 to 11.

17. The use of a protease in the production or sterilization of a pharmaceutical preparation or food-stuff in order to reduce the content of virus, pro-virus or virus-infected cells therein.

18. The use of a protease in the sterilization or decontamination of a surgical device in order to reduce contamination thereof with virus, provirus or virus-infected cells.

19. The use as claimed in Claim 17 wherein the pharmaceutical preparation is injectable.

20. The use as claimed in Claim 19 wherein the injectable preparation comprises whole blood or a blood fraction or component.

21. The use as claimed in Claim 17 wherein the pharmaceutical preparation comprises an animal tissue for transplantation.

22. The use as claimed in any of Claims 15 to 21 wherein said viral infection or contamination is an infection or contamination with a DNA or RNA-containing virus or with a prion.

23. A method according to Claim 22 wherein said viral infection or contamination is an infection with an enveloped virus.

24. A method according to Claim 23 wherein the enveloped virus is HIV or influenza virus.

25. A method of producing inactivated or attenuated virus for incorporation into a vaccine or vaccine component for providing a protective effect against viral infection in humans or animals, which comprises contacting virus with a protease.

26. Inactivated or attenuated virus produced by the method of Claim 23.

27. A vaccine comprising a virus according to Claim 24 and a pharmaceutically acceptable carrier.

European Patent Office

**PARTIAL EUROPEAN SEARCH REPORT**
which under Rule 45 of the European Patent Convention
shall be considered, for the purposes of subsequent
proceedings, as the European search report

Application number

EP 89309061.3

page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | PATENT ABSTRACTS OF JAPAN vol. 6, no. 239 (C-137) (1117), 26 November 1982; & JP- A - 57 140728 (MOCHIDA S.K.K.) 31.08.1982 <br> * the whole document * | 15-27 | A61K37/54 <br> A61K37/547 |
| X,P | PATENT ABSTRACTS OF JAPAN vol. 12, no. 353 (C-530) (3200), 21 September 1988; & JP - A - 63 109795 (TAKEDA) 14.05.1988 <br> * the whole document * | 15-27 | |
| X | CHEMICAL ABSTRACTS vol. 72, no. 15, 13 April 1970, page 98, column 2, abstract no. 87224j, Columbus, Ohio, USA; H. YOSHII: "Inhibition of viral infection by some protein compounds applied to the host before or after inoculation with some plant viruses" & Nippon Shokubutsu Byori Gakkaiho 1969, vol. 35, no. 4, pages 319-321 <br> * abstract * | 15-27 | TECHNICAL FIELDS SEARCHED (Int. Cl 4) <br><br> A61K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 15-27
Claims searched incompletely:
Claims not searched: 1-14
Reason for the limitation of the search:

Method for treatment of the human or animal body by therapy Article 52(4) EPC

| Place of search | Date of completion of the search | Examiner |
|---|---|---|
| Berlin | 08.11.1989 | P. . AVEDIKIAN |

EPO Form 1505.1 03 82'

| Category | Citation of document with indication, where appropriate, of relevant passages | Relevant to claim | CLASSIFICATION OF THE APPLICATION (Int. Cl.⁴) |
|---|---|---|---|
| A,P | GB - A - 2205746 (L. MAITLAND MC EWAN) <br> * the whole document * | 15-27 | |
| A,P | EP - A - 0313346 (GENZYME CORP.) <br> * the whole document * | 15-27 | |
| A | EP - A - 0250335 (INSERM) <br> * the whole document * | 15-27 | |
| A | WO - A - 8402846 (ADVANCED DRUG TECHNOLOGY) <br> * the whole document * | 15-27 | TECHNICAL FIELDS SEARCHED (Int. Cl.⁴) |